# EUROPEAN PATENT APPLICATION

(11) **EP 0 939 133 A2**
(43) Date of publication of application: **01.09.1999**
(21) Application number: 99102168.4
(22) Date of filing: 03.02.1999
(51) Int. Cl.: C12P 7/18

(54) **Method for producing polyol by recycling microorganisms**

(30) Priority: 03.02.1998 JP 3537998
(71) Applicant: Nikken Chemicals Company, Limited, Tokyo 104-0045 (JP)
(72) Inventor: Nishita, Kiyotaka, c/o Omiya Research Laboratory, Omiya-shi, Saitama 330-0835 (JP); Uetake, Noriko, c/o Omiya Research Laboratory, Omiya-shi, Saitama 330-0835 (JP)
(74) Representative: VOSSIUS & PARTNER

(57) **Abstract**

The invention provides an industrially advantageous method for producing polyol by recycling microorganisms, comprising repeating a process of separating and recovering microorganisms from a culture liquid after polyol production and a process of adding the separated and recovered microorganisms to a fresh culture medium for fermentation to produce polyol again, thereby recycling the separated and recovered microorganisms, wherein the nutrient ingredients in the culture medium for the second cycle of fermentation are a carbon source only, or wherein other nutrient ingredients in addition to the carbon source are adjusted to concentrations lower then the concentrations in the culture medium of the first cycle of fermentation.

## Description

The present invention relates to a method for producing polyol by microbial fermentation

It has been known as polyol-producing microorganisms that microorganisms belonging to the genus Torulopsis produces glycerin (Japanese Patent Publication No. 47-8589 and No. 63-9833); that microorganisms belonging to the genus Candida, Saccharomyces or Torulopsis (Japanese Patent Publication No. 47-20394 or No. 53-6231), microorganism belonging to the genus Brettanomyces (Japanese Patent Publication No. 63-9834), Pichia (Japanese Patent Publication No. 57-26114) or Rohnella (Japanese Patent Kokai No. 2-69186) produce arabitol; and additionally that microorganisms belonging to the genus Monilliella (Japanese Patent Kokai No. 60-110295). Aureobasidium (Japanese Patent Publication No. 61-31091) or Trichosporonoides WO97/26323) produce erythritol. Other than those described above, additionally, numerous microorganisms having the ability of producing mannitol, sorbitol, xylitol, etc., have been known.

Batch-wise fermentation processes and continuous fermentation processes have been known as fermentation processes of producing desired substances by utilizing microorganisms. However, operations for the continuous fermentation process are laborious in terms of stability during continuous use of the microorganism. Accordingly, the batch-wise fermentation process is generally practiced as a method for producing polyol at an industrial scale.

The batch-wise fermentation process comprises primarily culturing a microorganism at a small scale for a seed for main inoculating and culturing the seed in a large-scale fermentor charged with a culture medium containing substrates as the raw materials for main culturing. The culturing is terminated just when the concentration of polyol as the objective substance reaches its peak or just when the raw material substrates are consumed. By subsequently separating the microorganism from the culture broth, polyol is purified and recovered from the culture broth after the microorganism is separated. The microorganism thus separated is disposed.

Japanese Patent Kokai No. 5-137585 discloses a method for producing erythritol, using erythritol-producing microorganisms, by continuous culture of said microorganisms while maintaining the nitrogen containing rate in the microorganism at 2.5 to 4.5%.

Japanese Patent Kokai No. 8-23989 discloses a method for producing oligosaccharides containing a high concentration of panose, comprising contacting a microorgainsm belonging to the genus Aureobasidium with a sugar solution containing maltose as the main component, and additionally discloses a method for producing oligosaccharides repeatedly, comprising again contacting the microorganism separated from the culture broth after main culturing with the sugar solution. Other than the oligosaccharide, then, erythritol is produced as a by-product at a yield of about 10 %.

Fermentation production by a batch-wise microbial fermentation process is disadvantageous since a period of time is needed for the growth of the microorganism so as to allow the microorganism to produce an desired substance and since substrate components serving as energy sources for the microbial growth are also required, so that a longer period of time and larger of raw material substrates than those just required for the production of the desired substance are required.

A large amount of the microorganism increased at the termination of culturing is sterilized and inactivated for disposal although the microorganism still retains a sufficient ability of producing the desired substance, and the disposal costs are economically disadvantageous. Alternatively, according to the batch-wise fermentation process, a seed necessary for subsequent fermentation production should be prepared freshly each time. Thus, the seed culture in god timing to main culturing is laborious for production management.

Although continuous fermentation is said to be an effective method to overcome said problems, a particular additional equipment should be mounted because the equipment for production is not always the same as that of batch fermentation,, Accordingly, if batch fermentation is operated, it will be costly to mount a new additional equipment for continuous fermentation.

This, it is desirable to develop a method for producing polyol with enhanced productivity while effectively utilizing batch fermentation equipment presently used.

It is an object of the present invention to provide a method for industrially producing polyol at a high concentration, characterized in that the method can reduce the burden of seed culture on the procedures for polyol production by batch-wise culture, the disposal of a large amounts of microorganisms, and can cut down as much as possible the costs for equipment to be newly mounted.

The present inventors have found that polyol can efficiently be produced by recycling microorganisms, wherein such process repeats the steps of; separating and recovering the microorganism from the medium after producing polyol, culturing the said microorganism in a new medium containing as the nutrient ingredient either only a carbon source or other nutrient ingredients in addition to the carbon source at a concentration lower than that in the culture medium for the first cycle; again producing polyol; and collecting the polyol.

More specifically, the present invention relates to a method for producing polyol by recycling a microorganism which comprises the steps of (1) culturing a microorganism having the ability of producing polyol in a medium containing a carbon source and other nutrient ingredients for producing polyol; (2) collecting the accumulated polyol; (3) separating and recovering the microorganism from the culture media; (4) then culturing said microorganism in a fresh media containing, as a nutrient, carbon source only or other nutrient ingredients in addition to the carbon source in lower concentrations than those in the medium of said step (1) with the carbon source; (5) collecting the accumulated polyol; and then repeating said steps (3) to (5),

In one preferred embodiment of the invention, a method for producing erythritol by aerobic fermentation of Trichosporonoides megachilliensis SN-G42 (FERM BP-1430) is provided, comprising the steps of separating and recovering the microorganism from the culture medium after erythritol production by filtration or centrifugation; culturing the said microorganism in a fresh culture medium containing either glucose only or other nutrient ingredients in addition to glucose at low concentrations compared with a medium of the first cycle with glucose, and then repeating the said steps.

The invention will now be described in detail hereinbelow.

Any microorganism with the ability of producing polyol may satisfactorily be used, with no specific limitation, in accordance with the present invention; microorganisms producing erythritol include for example microorganisms belonging to the genus Aureobasidium described in Japanese Patent Kokai No. 63-9831; microorganisms belonging to the genus Trichosporonoides described in Japanese Patent Kokai No. 9-154589 or WO97/26323; microorganisms belonging to the genus Monilliella described in Japanese Patent Kokai No. 60-110295 or No. 9-154589; microorganisms belonging to the genus Yerobia, Ustilago, Phyllobadium or Trigonopsis described in Japanese Patent Kokai No. 9-154590; microorganisms belonging to the genus Trigonopsis or Candida described in Japanese Patent Publication No. 47-41549; microorganisms belonging to the genus Candida, Torulopsis, Hansenula, Pichia or Debariomyces described in Japanese Patent Publication No. 51-21071; microorganisms belonging to the genus Hanseniaspora, Saccharomyces, Kloechella, Kluveromyces or Torulaspora described in Biotechnol. Lett. , 7(4), 119-234 (1985); microorganisms belonging to the genus Aspergillus (S. A. Barer, J. Chem. Soc., 2538-2586(1958)); microorganisms belonging to the genus Eupenicillium, Monascus, Penicillium or Berzicillium described in Experimental Micology, Gaby. E., 4, 160-170(1980); microorganisms belonging to the genus Zigopichia (the genus is currently classified into genus Yamadajima) (Suzuki, Japanese Journal of Fermentation and Industry, 35(6), 1977); and microorganisms belonging to the genus Rhodotolura (J. Gen Microbiol., 18, 269(1985)).

Microorganisms having the ability of producing glycerin include microorganism belonging to the genus Toluropsis described in Japanese Patent Publication No. 47-8589 or No, 63-9833.

Microorganisms having the ability of producing arabitol include microorganisms belonging to the genus Candida, Saccharomyces or Torulopsis described in Japanese Patent Publication No. 47-20394 or No, 53-6231; microorganisms belonging to the genus Brettanomyces described in Japanese Patent Publication No. 63-9834; microorganisms belonging to the genus Pichia described in Japanese Patent Publication No. 57-26114; and microorganisms belonging to the genus Rohnella described in Japanese Patent Kokai No. 2-69186. Additionally, microorganisms having the ability of producing mannitol, sorbitol, xylitol, etc., are also illustrated.

In accordance with the present invention, preferably, use is made of the Trichosporonoides megachilliensis strain SN-G42 (FERM BP-1430, deposited under the old name as Aureobasisium strain SN-G42).

In accordance with the present invention, the culture medium for culturing such microorganisms and producing polyol generally comprises nutrient ingredients such as a carbon source, a nitrogen source and inorganic salts. The carbon source includes sacharides, such as glucose, fructose, etc., and carbohydrate containing these saccharides, such as starch-saccharified liquor, sweet potato syrup and beet syrup; and the carbon source preferably used is glucose. The sugar concentration in the culture medium is within the range of 20 to 60% (w/v), preferably 30 to 40 % (w/v).

As the nitrogen source, use is made of inorganic nitrogen compounds or organic nitrogen compounds that can be utilized by the microorganism, including for example yeast extract, peptone, corn steep liquor, ammonia, ammonium salts, urea, various amino acids, thiamin, biotin, etc.

As the inorganic salts, use is made of inorganic salts such as salts of phosphoric acid, magnesium, potassium, calcium and iron.

In addition to the aforementioned nutrient ingredients, furthermore, supplementary components for microbial growth, such as vitamin, may be added to the culture medium.

In case of culturing in a liquid medium, it is preferable to add defoaming agents, pH adjusting agents and buffers. As the defoaming agent, use is made of inorganic or organic defoaming agents, including for example polyvinyl alcohol, sorbitan fatty acid ester, and silicon-based compounds; the pH adjusting agents include for example sodium hydroxide, potassium hydroxide, sulfuric acid, hydrochloric acid, and nitric acid; and the buffers include for example sodium lactate, and various phosphates.

In accordance with the invention, the medium for a seed culture prior to main culturing as well as the medium for seed inoculation for the first cycle of main culturing contains essential nutrient ingredients, such as a carbon source, a nitrogen source and inorganic salts, as described above. When the concentration of the nutrient ingredients in the medium for the second cycle and subsequent cycles of main culturing is identical to that of the nutrient ingredients in the culture medium for the first cycle of main culturing, the polyol productivity is lowered. However, when the nutrient ingredients in addition to the carbon source in the medium for the second cycle and subsequent cycles of main culturing have a lower concentration than that of the individual nutrient ingredients in the medium of the first cycle of main culturing or when the medium for the second cycle and thereafter preferably contain only a carbon source, such is glucose, the polyol productivity can be elevated to the same level or to a higher level than the level obtained during the first cycle of main culturing.

In accordance with the invention, microorganisms are expected to exert two actions, namely growth and polyol production, during the first cycle of the main culturing. During the fermentation of the second cycle and the subsequent cycles of main culturing, however, it is not necessary to expedite growth of microorganism as using the microorganisms separated and recovered from the main culture at the first cycle, and the microorganism is required to concentrate upon polyol production,

In the present invention, aerobic fermentation is preferably conducted, and when the concentration of the nutrient ingredients in the culture medium in the second and subsequent cycles of the main culturing are the same as that of the nutrient ingredients in the culture medium of the first cycle of main culturing, the microorganism still grows, so that the polyol productivity is lowered

The reason why productivity is lowered is that when the microorganisms are excessive in the culture media, the medium is at a state of oxygen deficiency, so that the aerobic culture media conditions shift to anaerobic condition resulting in polyol production being possibly suppressed, since the polyol production under aerobic reaction

Therefore, as a means for preventing polyol productivity from being lowered, it is considered that the aerobic conditions for the second and subsequent cycles of main culturing is better than that of the first cycle of main culturing so as to maintain aerobic conditions for excessive microorganisms in the culture medium. Or it is either considered that the amounts of the nutrient ingredients to be added to the culture medium in the second and subsequent cycles for main culturing are regulated, compared with the amounts thereof for the first cycle of main culturing in order to inhibit the growth of the microorganism.

The method for restricting the additional amount of the nutrient ingredients according to the present invention is preferable in view of economy compared with the method for maintaining aerobic condition, because the costs for preparing the culture medium can be lowered.

The present invention can be applied to all the methods for producing fermentation products by utilizing microorganism. and is particularly effective for producing fermentation products, such as polyols produced by means of aerobic fermentation,

The step of separating and recovering microorganism is conducted by an apparatus that rapidly separates the culture liquid from the microorganisms, such as filtration with diatomaceous earth utilizing filter press, precision filtration, ultrafiltration and ceramic filtration using an organic membrane separator and ceramic separator; centrifugation using a batch-wise centrifuge or a continuous centrifuge; or precipitation processes comprising leaving the culture liquid after polyol production to stand alone for 4 to 6 hours to precipitate the microorganisms therein and suctioning the microorganism by means of a pump, thereby recovering the microorganisms. Filtration processes and centrifugations with a shorter processing time are preferred. Furthermore, the separated and recovered microorganisms are preferably stored in the cold while the microorganisms are prevented from drying.

The total amount of the separated and recovered microorganisms may be added to a subsequent culture media When the microbial polyol productivity is gradually reduced due to frequent microbial recycling or when it is difficult to transfer the total amount of separated and recovered microorganism into a subsequent fermentor for main culturing, a lower microbial concentration than the optimum for polyol production is added to a subsequent fermentor for main culturing and the microorganisms are made to grow in the medium therein, thereby adjusting the concentration of the microorganisms with a high polyol productivity to an optimum level for polyol production. In this case. nutrient ingredients essential for the growth, in addition to carbon sources, should necessarily be added to the culture medium. When the amounts of the nutrient ingredients are adjusted to concentrations similar to those during the first cycle of main culturing, the microorganism grows at an excess level. Accordingly, the nutrient ingredients in addition to the carbon source should be adjusted to low concentrations, compared with the concentrations for the first cycle of main culturing, so that the microbial concentration might be kept at an optimum concentration,

The method for producing polyol in accordance with the present invention comprises culturing a microorganism having the ability of producing polyol at a small scale to prepare a seed for main culturing, inoculating the seed into a large-scale fermentor charged with a liquid medium containing nutrient ingredients essential for the microbial growth and polyol production, and thereafter culturing the microorganism under aerated agitation conditions. The culturing is terminated by stopping the aerated agitation just when the polyol concentration reaches the peak, the microorganism is separated and recovered from the culture and culture liquid after separation of microorganism is purified, thereby obtaining polyol.

In the second and subsequent cycles of main culturing, the separated and recovered microorganism is added to a liquid medium containing nutrient ingredients in addition to the carbon source at concentrations lower than in the first cycle of main culturing or to a liquid medium preferably only containing a carbon source, such as glucose, etc., to re-start the culturing of the microorganism therein so as to produce polyol.

Thereafter, without preparing a new seed for main culturing, polyol is produced, by recycling the microorganism in a manner comprising repeating a process of separating and recovering the microorganism from the culture liquid after polyol production and a process of adding the separated and recovered microorganism to a freshly prepared liquid medium to produce polyol.

The optimum culturing conditions of pH, temperature, aeration ratio in case of aerobic fermentation, etc., vary, depending on the microorganism used, but the conditions for the batch-wise culturing thereof are not necessarily changed Additionally, the period of time for microbial growth is generally needed for batch-wise culturing, but in accordance with the present invention, such period is not necessary. Hence, the culturing time can be shortened. Culturing may satisfactorily be terminated just when the polyol concentration reaches the peak or the carbon source is consumed, and the timing can be determined appropriately by assaying the polyol concentration by known methods, such as high-performance liquid chromatography (abbreviated as HPLC) or thin-layer chromatography (abbreviated as TLC).

Any method for terminating the culturing of microorganism may be satisfactory, without limitation, as long as the method does not damage the microorganism; such methods include, for example, adjustment of aeration ratio and the lowering of the culturing temperature. When culturing is facilitated under aerobic conditions, the culturing can be terminated by stopping aeration,

According to the present invention, the seed culture can be omitted by repeatedly utilizing the microorganism without requiring any specific apparatus, while said seed culture is inevitable for batch-wise fermentation Moreover, the fermentation period can be shortened and also the amount of waste microbial cells can be decreased. The method of the present invention is industrially advantageous since the nutrient ingredients to be added to a medium for the second and subsequent cycles can be reduced.

### EXAMPLES

The present invention is now described in more detail with reference to the embodiments below, but the invention is not limited to these embodiments.

### Example 1

### 1. Seed culture:

40 ml of a culture medium containing glucose at 30 w/v % (w/v % is abbreviated as % hereinafter) and yeast extract at 1 % as nutrient ingredient were charged in a 500-ml Erlenmeyer flask, which was then sealed with a cotton plug, for 20-min sterilization at 120°C in an autoclave. Trichosporonoides megachilliensis SN-G42 (FERM BP-1430) was inoculated into the medium. The microbial strain was rotation cultured at 35°C with agitation at 220 rpm for 3 days. The resulting culture medium was used as the seed culture medium (A).

### 2. First cycle of fermentation:

3 liters of a culture media containing glucose at 40 %, yeast extract at 1 % as nutrient ingredient, and a defoaming agent (Adecanol LG109; manufactured by Asahi Denka Industry, Co. Ltd.) were placed and sterilized in a 7-liter fermentor (Model MD-C; manufactured by Iwashiya, Co.), followed by addition of the seed culture medium (A). The culture was conducted at 35°C while agitating at 680 rpm and passing air therethrough at a feed rate of 1.5 liters/minute for 4 days. Just when the glucose was consumed , the culturing was terminated.

### 3. Second cycle of fermentation:

From the culture liquid after the completion of the first cycle of fermentation were separated the microorganism with a centrifuge (PS-18IV manufactured by Tomy, Co. Ltd.) under the condition of a centrifugal force ×5000G, and the resulting microorganism was added to a 7-liter fermentor containing 3 liters of a culture medium containing 40 % glucose, and cultured under the same conditions as for the first cycle of fermentation. Then, the glucose was fully consumed in 3 days. Thus, the culturing was terminated.

Erythritol produced during the first and second cycles of fermentation was assayed by HPLC. In the first cycle of fermentation, the erythritol concentration was 168 g/liter. In the second cycle of fermentation, the erythritol concentration was 200 g/liter. Furthermore, the second cycle of fermentation required less days.

The total amount of the microorganism separated from the first cycle of fermentation was recycled in the second cycle of fermentation. Compared with the case of carrying out the conventional batch-wise fermentation process carried out twice, the amount of waste microbial cells was decreased to 1/2.

### Example 2

### 1. Seed culture:

50 ml of a culture medium containing glucose at 30 % and yeast extract at 1 % were charged in a 500-ml Erlenmeyer flask, which was then sealed with a cotton plug, for 20-min sterilization at 120°C in an autoclave. Trichosporonoides megachilliensis SN-G42 (FERM BP-1430) was inoculated into the medium. The microbial strain was rotary cultured at 35°C with agitation at 220 rpm for 3 days. The resulting culture medium was used as a seed culture medium (B).

### 9. First cycle of fermentation:

3 liters of a culture medium containing glucose at 40 %, yeast extract at 1 % and a defoaming agent (Adecanol LG109; manufactured by Asahi Denka Industry, Co. Ltd.) were placed and sterilized in a 7-liter fermentor (Model MD-C; manufactured by Iwashiya, Co.), followed by addition of the seed culture medium (B). The culture was conducted at 35°C while agitating at 680 rpm, and passing air therethrough at a speed rate of 1.5 liters/minute for 3 days.

### 3. Second cycle of fermentation:

300 ml of the culture liquid after the completion of the first cycle of fermentation were separated from the microorganisms with a centrifuge (PS-18IV manufactured by Tomy, Co. Ltd.) 5000G, and the amount of wet microorganisms was determined. 13 ml each of said culture medium containing glucose at 40%, yeast extract at 1%, 0.5% or 0.25% as the nitrogen source, vitamin source and mineral source was then charged into a sterilized 500 ml Erlenmeyer flask, and was inoculated with wet microorganisms in the same concentration of the microorganisms as that at the termination of the first cycle of fermentation, and was cultured at 35 °C for 2 days while agitating at 220 rpm, In this case of culturing in the Erlenmeyer flask, sodium lactate was added in order to prevent lowering of pH.

Erythritol produced during the first and second cycles of fermentation was assayed by HPLC; at the first cycle of fermentation, the erythritol concentration was 140 g/liter; the result of the second cycle of fermentation in each concentration of yeast extract as nutrient ingredient is shown below.

| Concentration of yeast extract (%) | Concentration of erythritol (g/L) |
|---|---|
| 1 | 139.8 |
| 0.5 | 139.5 |
| 0.25 | 152.8 |

## Claims

1. A method for producing polyol by recycling a microorganism, said method comprising the steps of (1) culturing a microorganism having the ability of producing polyol in a medium containing a carbon source and other nutrient ingredients for producing polyol; (2) collecting the accumulated polyol; (3) separating and recovering the microorganism from the culture medium; (4) then culturing the microorganism in a fresh medium containing, as a nutrient, a carbon source only or other nutrient ingredients in addition to the carbon source at a concentration lower than that in the medium of said step (1); (5) collecting the accumulated polyol; and then repeating said steps (3) to (5).

2. The method of claim 1, wherein the production of polyol is carried out under aerobic condition.

3. The method of claim 1 or 2, wherein the microorganism has the ability of producing erythritol and the main component of the produced polyol is erythritol.

4. The method of any one of claims 1 to 3, wherein the microorganism belongs to the genus Trichosporonoides.

5. The method of claim 4, wherein the microorganism is strain Trichosporonoides megachilliensis SN-G42 (FERM BP-1430).

6. The method of any one of claims 1 to 5, wherein the carbon source is glucose.

7. The method of any one of claims 1 to 6, wherein the separating and recovering of the microorganism is carried out by filtration with diatomaceous earth utilizing a filter press, precision filtration, ultrafiltration, ceramic filtration or centrifugation.
